# EUROPÄISCHE PATENTSCHRIFT

(11) **EP 3 751 594 B1**
(45) Veröffentlichungstag und Bekanntmachung des Hinweises auf die Patenterteilung: **28.08.2024**
(21) Anmeldenummer: 19179314.0
(22) Anmeldetag: 11.06.2019
(51) Int. Cl.: H01J 35/08

(54) **RÖNTGENRÖHRE**
X-RAY TUBE
TUBE A RAYONS X

(43) Veröffentlichungstag der Anmeldung: 16.12.2020
(73) Patentinhaber: Siemens Healthineers AG, 91301 Forchheim (DE)
(72) Erfinder: Freudenberger, Jörg, 90562 Kalchreuth (DE); Fritzler, Anja, 91052 Erlangen (DE); Geithner, Peter, 91058 Erlangen (DE); Radicke, Marcus, 90587 Veitsbronn (DE)
(74) Vertreter: Siemens Healthineers Patent Attorneys

(56) Entgegenhaltungen:
- DE-A1- 2 719 609
- US-A1- 2004 120 463
- US-B1- 6 487 272

## Beschreibung

Die Erfindung betrifft eine Röntgenröhre, eine Röntgeneinrichtung und eine Mammographieeinrichtung.

Für eine bildgebende Untersuchung mit Röntgenstrahlung wird üblicherweise eine Röntgenröhre verwendet. Eine Röntgeneinrichtung weist typischerweise die Röntgenröhre und einen Röntgendetektor zur Durchführung der bildgebenden Untersuchung auf. Die bildgebende Untersuchung kann insbesondere eine bildgebende Mammographieuntersuchung, insbesondere ein Mammographie-Screening, sein. Idealerweise ermöglicht die Röntgenröhre die bildgebende Untersuchung mit einer hohen Bildqualität und gleichermaßen mit einer möglichst niedrigen Röntgenstrahlendosis, vorzugsweise durch eine Anpassung eines Emissionsspektrums der Röntgenstrahlung. Vorzugsweise ist die Röntgenstrahlung bzw. das Emissionsspektrum, insbesondere deren Energieverteilung, in Hinblick auf einen maximalen Bildkontrast abgestimmt. Diesbezüglich ist bekannt, dass die Röntgenstrahlendosis desto geringer ist, je monochromatischer die Röntgenstrahlung bzw. das Emissionsspektrum ist.

Beispielsweise wird herkömmliche Röntgenstrahlung mittels eines Filters angepasst, wobei gleichermaßen die Bildqualität und die Röntgenstrahlendosis optimiert werden soll. Üblicherweise verringert der Filter die für die Bildqualität nützliche Röntgenstrahlung, wodurch zum Ausgleich eine Intensität der Röntgenstrahlung erhöht werden muss. Eine weitere Möglichkeit der Anpassung des Emissionsspektrums ist, dass die Röntgenstrahlung generierende Anodenmaterial zu variieren.

Aus der noch nicht veröffentlichen Anmeldung EP 18196848.8 ist eine Röntgenröhre mit einer zumindest abschnittsweise periodisch alternierenden Oberflächenstruktur der Anode bekannt.

Die US 2019 / 0 030 363 A1 offenbart zwei zueinander beabstandete Anodenmaterialien, wobei das erste Anodenmaterial Bremsstrahlung generiert und das zweite Anodenmaterial monochromatische Röntgenstrahlung mittels der Bremsstrahlung emittiert.

Aus US 6,487,272 B1 ist eine Röntgenröhre bekannt, wobei ein Elektronstrahl im Wesentlichen senkrecht auf eine Wolframbasierte Transmission-Anodenschicht auftrifft und die generierte Röntgenstrahlung vor dem Austritt durch ein Beryllium-Fenster eine Zwischenschicht zur Verringerung einer Abblätterung der Anodenschicht vom Fenster durchdringt.

Die US 2004 / 0 120 463 A1 offenbart eine DrehanodenRöntgenröhre mit einer Anodenschicht zur Generierung von Röntgenstrahlung, wobei die Anodenschicht innerhalb mehrerer periodisch entlang der Oberfläche der Anode verteilter Kerben angeordnet ist.

DE 27 19 609 A1 beschreibt eine Röntgenröhre mit einer mehrschichtigen Anode und einer Elektronenquelle, wobei die Röntgenstrahlung in einem engen Raumwinkel antiparallel zur Richtung der einfallenden Elektronen austritt.

Der Erfindung liegt die Aufgabe zu Grunde, eine Röntgenröhre, eine Röntgeneinrichtung und eine Mammographieeinrichtung anzugeben, bei welchen ein Anteil an monochromatischer Röntgenstrahlung erhöht wird.

Die Erfindung wird durch die Merkmale des unabhängigen Anspruchs 1 definiert. Vorteilhafte Ausgestaltungen sind in den Unteransprüchen beschrieben.

Die erfindungsgemäße Röntgenröhre weist
- einen einen Elektronenstrahl emittierenden Elektronenemitter und
- eine mehrschichtige Anode auf,
- wobei die mehrschichtige Anode eine dem Elektronenstrahl zugewandte erste Anodenschicht und eine dem Elektronenstrahl abgewandte zweite Anodenschicht aufweist,

- wobei die erste Anodenschicht ein erstes Anodenmaterial zur Generierung einer Bremsstrahlung mittels des auftreffenden Elektronenstrahls aufweist,
- wobei das zweite Anodenmaterial ein Konvertermaterial ist,
- wobei die zweite Anodenschicht ein zweites Anodenmaterial zur Generierung einer weiteren Röntgenstrahlung mittels der Bremsstrahlung aufweist,
- wobei die weitere Röntgenstrahlung monochromatischer ist als die Bremsstrahlung,
- wobei die erste Anodenschicht und die zweite Anodenschicht flächig aneinander angrenzen,
- wobei die erste Anodenschicht gemeinsam mit der zweiten Anodenschicht eine Anodenmikrostruktur bildet,
- wobei die Anodenmikrostruktur entlang einer Oberfläche der mehrschichtigen Anode periodisch wiederkehrend angeordnet ist,
- wobei der Elektronenstrahl auf der Oberfläche in einem Einfallwinkel kleiner 90° auftrifft und
- wobei der Einfallwinkel zwischen 10° und 60° liegt.

Die Röntgenröhre ist insbesondere vorteilhaft, weil aufgrund der räumlichen Nähe der ersten Anodenschicht zur zweiten Anodenschicht mehr Bremsstrahlung die zweite Anodenschicht durchdringt, wodurch vorzugsweise ein Anteil an monochromatischer Röntgenstrahlung im Emissionsspektrum der Röntgenröhre erhöht ist. In anderen Worten geht weniger Bremsstrahlung außerhalb des zweiten Anodenmaterials verloren. Ein weiterer Vorteil kann sein, dass die zweite Anodenschicht vorzugsweise nicht von Primärelektronen, sondern von Sekundärelektronen getroffen wird. Vorzugsweise kann eine Bildqualität und/oder eine Röntgenstrahlendosis bei einer bildgebenden Untersuchung eines Patienten optimiert werden. Beispielsweise kann die bildgebende Untersuchung eine bildgebende Mammographieuntersuchung, eine Durchleuchtung, eine Angiographie, eine Computertomographie und/oder eine kontrastmittelgestützte Untersuchung umfassen.

Die Röntgenröhre kann insbesondere gemäß einer Stehanodenröntgenröhre, einer Drehanodenröntgenröhre oder einer Drehkolbenröntgenröhre ausgebildet sein. Typischerweise ist die Röntgenröhre evakuiert. Der Elektronenstrahl weist typischerweise mehrere Elektronen auf, welche von dem Elektronenemitter zu der mehrschichtigen Anode hin vorzugsweise beschleunigt werden. Der Elektronenstrahl wird beispielsweise von einer Ablenkeinheit, insbesondere von einer magnetischen Ablenkeinheit, der Röntgenröhre auf die mehrschichtige Anode ausgerichtet. Der Elektronenemitter kann insbesondere einen Wendelemitter, einen Flachemitter oder einen Feldeffekt-Emitter aufweisen.

Die mehrschichtige Anode weist insbesondere mehrere Anodenschichten an unterschiedlichen Anodenmaterialien auf. Die erste Anodenschicht kann sich in einer Ausdehnung und/oder in einem Volumen und/oder in einem Gewicht von der zweiten Anodenschicht unterscheiden. Die erste Anodenschicht kann beispielsweise auf die zweite Anodenschicht aufgetragen werden oder umgekehrt. Das Auftragen kann insbesondere ein Sprühen, ein Gießen, ein Befestigen, ein Bedampfen, ein Sputtern, ein selektives elektrochemisches Beschichten und/oder ein Maskieren umfassen. Grundsätzlich ist es denkbar, dass nach dem Auftragen die erste Anodenschicht und/oder die zweite Anodenschicht zumindest teilweise wieder abgetragen wird, insbesondere wenn das Auftragen ohne Maskieren erfolgt. Durch das Auftragen grenzen die erste Anodenschicht und die zweite Anodenschicht flächig aneinander an. Typischerweise ist zwischen der ersten Anodenschicht und der zweiten Anodenschicht kein Material und/oder kein Abstand. Die erste Anodenschicht und die zweite Anodenschicht teilen insbesondere einen gemeinsamen flächigen Kontaktbereich. Der gemeinsame flächige Kontaktbereich kann zweidimensional sein, beispielsweise eben, oder dreidimensional verformt sein, beispielsweise wellenförmig oder L-förmig oder U-förmig. Wenn der gemeinsame flächige Kontaktbereich dreidimensional verformt ist, umschließt die erste Anodenschicht zumindest teilweise insbesondere die zweite Anodenschicht oder umgekehrt. Gemäß einer besonderen Ausführungsform umschließt die zweite Anodenschicht die erste Anodenschicht, insbesondere L-förmig oder U-förmig, weil dadurch mehr Bremsstrahlung auf der zweiten Anodenschicht auftreffen kann.

Dass die erste Anodenschicht dem Elektronenstrahl zugewandt und dass die zweite Anodenschicht dem Elektronstrahl abgewandt ist, bedeutet insbesondere, dass der Elektronenstrahl zunächst auf die erste Anodenschicht auftrifft. Die Primärelektronen des Elektronenstrahls treffen auf der ersten Anodenschicht auf. Die Primärelektronen sind solche Elektronen, welche noch nicht mit dem ersten Anodenmaterial oder dem zweiten Anodenmaterial wechselgewirkt haben. Elektronen, welche bereits eine Wechselwirkung mit dem ersten Anodenmaterial durchgeführt haben, sind Sekundärelektronen. Die Wechselwirkung kann ein Streuen, ein Fluoreszieren, ein Ablenken, ein Absorbieren und/oder ein Abschwächen sein. Typischerweise wird der Elektronenstrahl von der ersten Anodenschicht vollständig oder zumindest teilweise absorbiert und/oder abgeschwächt. Wenn der Elektronenstrahl teilweise absorbiert wird, kann der Elektronenstrahl nach der ersten Anodenschicht grundsätzlich auf die zweite Anodenschicht auftreffen. Die erste Anodenschicht ist insbesondere zwischen dem Elektronenemitter und der zweiten Anodenschicht angeordnet. Die mehrschichtige Anode ist relativ zum Elektronenstrahl derart ausgerichtet, dass der Elektronenstrahl zunächst auf der ersten Anodenschicht auftrifft.

Die erste Anodenschicht besteht aus dem ersten Anodenmaterial. Das erste Anodenmaterial ist insbesondere derart ausgebildet, dass aus den auftreffenden Elektronen Bremsstrahlung generiert werden kann. Das Generieren der Bremsstrahlung erfolgt durch ein derartiges Wechselwirken eines eintreffenden Elektrons mit einem Nukleus des ersten Anodenmaterials, dass das eintreffende Elektron abgelenkt und somit um eine Deltaenergie abgeschwächt wird, wobei ein Photon mit der Deltaenergie als Teil der Bremsstrahlung entsteht. Die Bremsstrahlung ist eine breitbandige und/oder kontinuierliche Röntgenstrahlung.

Die zweite Anodenschicht besteht aus dem zweiten Anodenmaterial. Das zweite Anodenmaterial ist ein Konvertermaterial. Das zweite Anodenmaterial ist derart ausgebildet, dass aus der vom zweiten Anodenmaterial absorbierten Bremsstrahlung die weitere Röntgenstrahlung generiert werden kann. Das Generieren der weiteren Röntgenstrahlung kann insbesondere durch ein Fluoreszieren des zweiten Anodenmaterials in Abhängigkeit eines eintreffenden Bremsstrahlungsphotons erfolgen. Alternativ oder zusätzlich können von dem ersten Anodenmaterial zurückgestreute Elektronen, insbesondere die Sekundärelektronen, auf das zweite Anodenmaterial treffen, wobei die weitere Röntgenstrahlung beispielsweise durch Fluoreszieren generiert wird. Die weitere Röntgenstrahlung kann insbesondere die Fluoreszenz-Photonen aufweisen. Eine Fluoreszenz-Rate des zweiten Anodenmaterials ist typischerweise höher als eine Fluoreszenz-Rate des ersten Anodenmaterials.

Das erste Anodenmaterial unterscheidet sich derart von dem zweiten Anodenmaterial, dass die weitere Röntgenstrahlung monochromatischer ist als die Bremsstrahlung. Das erste Anodenmaterial ist zur Generierung von Bremsstrahlung aus den Elektronen geeignet und das zweite Anodenmaterial ist zur Generierung der weiteren Röntgenstrahlung aus der Bremsstrahlung geeignet. Dass die weitere Röntgenstrahlung monochromatischer ist als die Bremsstrahlung bedeutet, dass ein Emissionsspektrum der Bremsstrahlung im Vergleich zu einem Emissionsspektrum der weiteren Röntgenstrahlung breitbandiger ist. Wenn die weitere Röntgenstrahlung monochromatischer ist, ist das Emissionsspektrum der weiteren Röntgenstrahlung typischerweise durch einen höheren Anteil an charakteristischer Röntgenstrahlung gekennzeichnet, insbesondere durch einen höheren Anteil von Photonen der K-Linien des zweiten Anodenmaterials im Vergleich zum Emissionsspektrum der Bremsstrahlung. Das Emissionsspektrum der weiteren Röntgenstrahlung kann insbesondere mehr und/oder stärkere charakteristische Linien aufweisen als das Emissionsspektrum der Bremsstrahlung.

Eine bevorzugte Ausführungsform sieht vor, dass sich das erste Anodenmaterial in einer charakteristischen Linie eines Emissionsspektrums von dem zweiten Anodenmaterial unterscheidet. Diese Ausführungsform ist insofern vorteilhaft, weil das erste Anodenmaterial zur Generierung der Bremsstrahlung optimiert sein kann und das zweite Anodenmaterial zur Generierung der weiteren Röntgenstrahlung optimiert sein kann.

Eine bevorzugte Ausführungsform sieht vor, dass das erste Anodenmaterial zumindest ein Material der folgenden Liste aufweist:
- Wolfram,
- Gold.

Diese Ausführungsform ist vorteilhaft, weil Wolfram besonders hitzebeständig ist und/oder Gold ein besonders vorteilhaftes Emissionsspektrum aufweist.

Eine bevorzugte Ausführungsform sieht vor, dass das zweite Anodenmaterial zumindest ein Material der folgenden Liste aufweist:
- Zinn,
- Silber,
- Molybdän,
- Palladium.

Diese Ausführungsform ist insbesondere vorteilhaft, weil ein Emissionsspektrum mit einer bestimmten Mischung der vorstehenden Materialien eine charakteristische Linie im Energiebereich zwischen 20 bis 34 keV aufweist, welche insbesondere für eine bildgebende Mammographieuntersuchung vorteilhaft sein kann. Grundsätzlich sind weitere Mischungen der Materialien denkbar, welche sich im jeweiligen Emissionsspektrum, insbesondere in deren charakteristischen Linien, unterscheiden.

Die erste Anodenschicht bildet gemeinsam mit der zweiten Anodenschicht eine Anodenmikrostruktur, wobei die Anodenmikrostruktur entlang einer Oberfläche der mehrschichtigen Anode periodisch wiederkehrend angeordnet ist und wobei der Elektronenstrahl auf der Oberfläche in einem Einfallwinkel kleiner 90° auftrifft. Der Elektronenstrahl trifft in einem spitzen Winkel auf die Oberfläche. Der Einfallwinkel liegt zwischen 10° und 60°, vorzugsweise zwischen 20° und 50° und kann besonders vorteilhafterweise 45° betragen. Der Einfallwinkel ist der Winkel zwischen dem Elektronenstrahl und einer, insbesondere makroskopischen, Oberfläche der mehrschichtigen Anode. Der Einfallwinkel ist insbesondere unabhängig von der Anordnung und/oder Ausbildung der Anodenmikrostruktur.

Die Anodenmikrostruktur kann kegelförmig sein, insbesondere wenn eine einzelne Anodenmikrostruktur der periodisch wiederkehrenden Anodenmikrostruktur im Schnitt dargestellt wird. Typischerweise bildet die einzelne Anodenmikrostruktur makroskopisch betrachtet eine Rille oder eine Welle. Insbesondere die periodisch wiederkehrende Anodenmikrostruktur bildet eine wellenförmige oder sinusförmige Oberfläche der mehrschichtigen Anode. In anderen Worten ist die Oberfläche der mehrschichtigen Anode typischerweise gerillt. Die Anodenmikrostruktur kann beispielsweise eine maximale Ausdehnung kleiner als 1 mm, vorzugsweise kleiner als 100 µm, besonders vorteilhafterweise kleiner als 10 µm aufweisen. Die maximale Ausdehnung kann beispielsweise einer Höhe des Kegels entsprechen. Dass die Anodenmikrostruktur periodisch wiederkehrend angeordnet ist, umfasst insbesondere ein mehrmaliges Replizieren der Anodenmikrostruktur entlang der Oberfläche. Die mehreren periodisch wiederkehrenden Anodenmikrostrukturen bilden insbesondere die wellenförmige oder sinusförmige Oberfläche der mehrschichtigen Anode. Diese Ausführungsform ist insbesondere vorteilhaft, weil der Elektronenstrahl und/oder die Bremsstrahlung und/oder die weitere Röntgenstrahlung insbesondere vor einem Austritt durch ein Röntgenstrahlungsaustrittfenster mehrmals mit der mehrschichtigen Anode, vorzugsweise mit der periodisch wiederkehrenden Anodenmikrostruktur wechselwirken kann. In dieser Ausführungsform ist vorzugsweise ein Anteil an monochromatischer Röntgenstrahlung durch die periodisch wiederkehrende Anodenmikrostruktur erhöht. Ein weiterer Vorteil kann sein, dass an einem Kegel der Anodenmikrostruktur zurückgestreute Elektronen auf einen weiteren Kegel der Anodenmikrostruktur auftreffen, um die weitere Röntgenstrahlung zu generieren. In anderen Worten wird der Anteil an monochromatischer Röntgenstrahlung dadurch erhöht, dass die mehrschichtige Anode mehrere Kegel, insbesondere Anodenschichten, mit dem zweiten Anodenmaterial zur Generierung der weiteren Röntgenstrahlung aufweist. Ein weiterer Vorteil kann sein, dass die dem Elektronenstrahl zugewandte Fläche der mehrschichtigen Anode, insbesondere des ersten Anodenmaterials vergrößert wird.

Eine bevorzugte Ausführungsform sieht vor, dass die mehrschichtige Anode eine dem Elektronenstrahl zugewandte dritte Anodenschicht und eine dem Elektronenstrahl abgewandte vierte Anodenschicht aufweist, wobei die dritte Anodenschicht ein drittes Anodenmaterial zur Generierung der Bremsstrahlung mittels des auftreffenden Elektronenstrahls aufweist, wobei die vierte Anodenschicht ein viertes Anodenmaterial zur Generierung der weiteren Röntgenstrahlung mittels der Bremsstrahlung aufweist, wobei die dritte Anodenschicht und die vierte Anodenschicht flächig aneinander angrenzen und wobei sich das zweite Anodenmaterial in einer charakteristischen Linie eines Emissionsspektrums von dem vierten Anodenmaterial unterscheidet. Die dritte Anodenschicht und die vierte Anodenschicht sind relativ zum Elektronenstrahl insbesondere neben der ersten Anodenschicht und der zweiten Anodenschicht angeordnet. Das zweite Anodenmaterial und das vierte Anodenmaterial können grundsätzlich die gleichen Materialien aufweisen und sich typischerweise in einer Mischung der Materialien unterscheiden. Alternativ ist es denkbar, dass das zweite Anodenmaterial ein Material aufweist, welches das vierte Anodenmaterial nicht aufweist und umgekehrt. Grundsätzlich kann das zweite Anodenmaterial teilweise dieselben charakteristischen Linien aufweisen wie das vierte Anodenmaterial. Das erste Anodenmaterial kann dem dritten Anodenmaterial entsprechen oder alternativ sich in einer charakteristischen Linie von dem dritten Anodenmaterial unterscheiden. Diese Ausführungsform ist insbesondere vorteilhaft, weil die mehrschichtige Anode ein Emissionsspektrum mit mehreren charakteristischen Linien bereitstellen kann. Vorzugsweise kann die Röntgenröhre daher für verschiedene bildgebende Untersuchungen verwendet werden.

Eine besonders vorteilhafte Ausgestaltung der vorherigen Ausführungsform ist, dass der Elektronenstrahl auf die erste Anodenschicht oder auf die dritte Anodenschicht ausrichtbar ist. Diese Ausführungsform ist insbesondere vorteilhaft, weil die mehrschichtige Anode verschiedene Emissionsspektren bereitstellen kann. Beispielsweise kann in diesem Fall eine bildgebende Untersuchung mit weiterer Röntgenstrahlung der zweiten Anodenschicht oder mit weiterer Röntgenstrahlung der vierten Anodenschicht durchgeführt werden. Alternativ oder zusätzlich kann gemäß einem Dual-Energy-Messprotokoll das Energiespektrum während der bildgebenden Messung alternierend festgelegt werden, insbesondere durch das Ausrichten des Elektronenstrahls auf die erste Anodenschicht oder auf die dritte Anodenschicht.

Eine Ausführungsform sieht vor, dass die Anodenmikrostruktur, insbesondere wie zuvor beschrieben, periodisch wiederkehrend angeordnet ist und ein erster Kegel der Anodenmikrostruktur die erste Anodenschicht sowie die zweite Anodenschicht aufweist und ein zweiter Kegel der Anodenmikrostruktur die dritte Anodenschicht sowie die vierte Anodenschicht aufweist. Diese Ausführungsform ist insofern vorteilhaft, weil eine Röntgenröhre mit einer kompakten mehrschichtigen Anode bereitgestellt wird, welche vorzugsweise verschiedene Emissionsspektren generiert.

Eine Röntgeneinrichtung weist die Röntgenröhre und einen Röntgendetektor auf. Der Röntgendetektor kann insbesondere die weitere Röntgenstrahlung nach einem Durchleuchten des Patienten erfassen. Mittels der erfassten weiteren Röntgenstrahlung kann vorzugsweise ein medizinisches Bild des Patienten rekonstruiert und/oder in einer Speichereinheit und/oder auf einer Anzeigeeinheit bereitgestellt werden.

Die Mammographieeinrichtung weist insbesondere die Röntgeneinrichtung und eine Brustlagerungsvorrichtung auf. Auf der Brustlagerungsvorrichtung kann vorzugsweise eine Brust des Patienten, beispielsweise einer Patientin, während der bildgebenden Mammographieuntersuchung gelagert werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleichbleibende Strukturen und Einheiten mit demselben Bezugszeichen wie beim erstmaligen Auftreten der jeweiligen Struktur oder Einheit benannt.

Es zeigen:
Fig. 1 eine Röntgenröhre in einem ersten Ausführungsbeispiel, das nicht unter den Schutzbereich des Anspruchs 1 fällt,
Fig. 2 eine mehrschichtige Anode in einem zweiten Ausführungsbeispiel,
Fig. 3 eine mehrschichtige Anode in einem dritten Ausführungsbeispiel und
Fig. 4 eine Röntgeneinrichtung in einem vierten Ausführungsbeispiel, das nicht unter den Schutzbereich des Anspruchs 1 fällt.

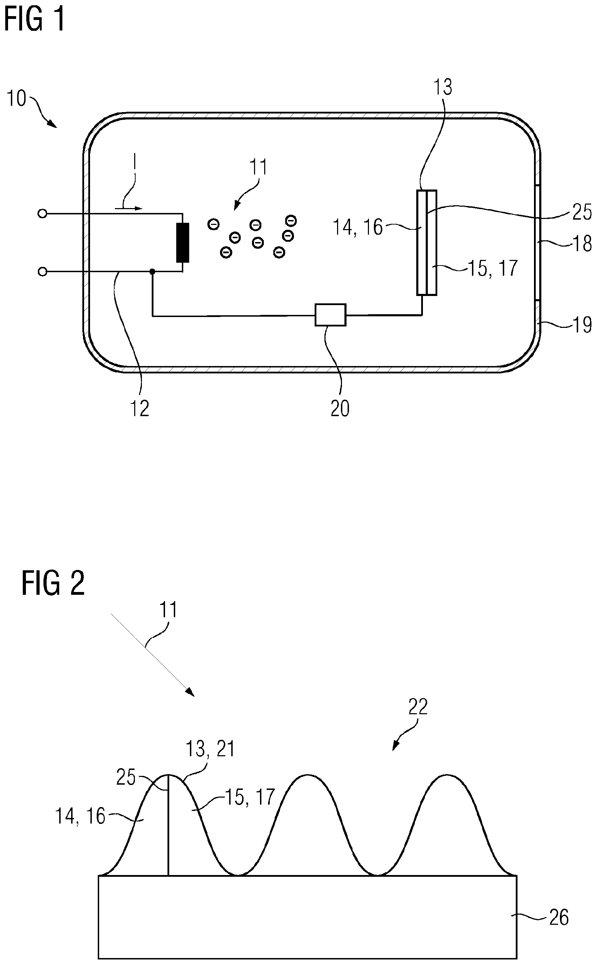

Fig. 1 zeigt eine Röntgenröhre 10 in einem ersten Ausführungsbeispiel. Die Röntgenröhre 10 weist einen einen Elektronenstrahl 11 emittierenden Elektronenemitter 12 und eine mehrschichtige Anode 13 auf. Der Elektronenemitter 12 emittiert den Elektronenstrahl 11 insbesondere während einer bildgebenden Untersuchung in Abhängigkeit von einem Heizstrom I. Die Elektronen des Elektronenstrahls 11 werden insbesondere von einer Spannungsversorgung 20 auf die mehrschichtige Anode 13 hin beschleunigt. Die Beschleunigungsspannung liegt typischerweise zwischen 20 und 150 keV.

Die mehrschichtige Anode 13 weist eine dem Elektronenstrahl 11 zugewandte erste Anodenschicht 14 und eine dem Elektronenstrahl 11 abgewandte zweite Anodenschicht 15 auf. Die erste Anodenschicht 14 und die zweite Anodenschicht 15 grenzen flächig aneinander an. Ein gemeinsamer flächiger Kontaktbereich 25 ist in diesem Ausführungsbeispiel zweidimensional, insbesondere eben.

Die erste Anodenschicht 14 weist ein erstes Anodenmaterial 16 zur Generierung einer Bremsstrahlung mittels des auftreffenden Elektronenstrahls 11 auf. Das erste Anodenmaterial 16 weist beispielsweise zumindest ein Material der folgenden Liste auf:
- Wolfram,
- Gold.

Die zweite Anodenschicht 15 weist ein zweites Anodenmaterial 17 zur Generierung einer weiteren Röntgenstrahlung mittels der Bremsstrahlung auf. Das erste Anodenmaterial 16 unterscheidet sich insbesondere von dem zweiten Anodenmaterial 17 in einer charakteristischen Linie eines Emissionsspektrums. Das zweite Anodenmaterial 17 weist beispielsweise zumindest ein Material der folgenden Liste auf:
- Zinn,
- Silber,
- Molybdän,
- Palladium.

Die weitere Röntgenstrahlung ist monochromatischer als die Bremsstrahlung. Die weitere Röntgenstrahlung kann beispielsweise durch ein Röntgenstrahlungsaustrittfenster 18 aus einem Gehäuse 19 der Röntgenröhre 10 austreten. Innerhalb des Gehäuses 19 ist typischerweise ein Vakuum.

**Fig. 2** zeigt die mehrschichtige Anode 13 in einem zweiten Ausführungsbeispiel. In diesem Ausführungsbeispiel ist der Elektronenstrahl 11 mit einem Pfeil gekennzeichnet.

Die erste Anodenschicht 14 bildet gemeinsam mit der zweiten Anodenschicht 15 eine Anodenmikrostruktur 21. Die Anodenmikrostruktur 21 ist entlang einer Oberfläche 22 der mehrschichtigen Anode 13 periodisch wiederkehrend angeordnet. Die Anodenmikrostruktur 21 ist kegelförmig, wenn einzeln im Schnitt betrachtet. Die periodische wiederkehrenden Anodenmikrostrukturen sind vorzugsweise derart nebeneinander angeordnet, dass sich das erste Anodenmaterial 16 und das zweite Anodenmaterial 17 abwechseln.

Der Elektronenstrahl 11 trifft auf der Oberfläche 22 in einem Einfallwinkel kleiner 90° auf. Die Oberfläche 22 ist wellenförmig und/oder sinusförmig. Eine Unterseite 26 der Oberfläche 22 der mehrschichtigen Anode 13 weist typischerweise Wolfram auf oder ein alternatives hitzebeständiges und/oder wärmeleitendes Kühlmaterial auf. Vorzugweise treffen die Elektronen des Elektronenstrahls 11 die erste Anodenschicht 14 und weniger bis gar nicht die zweite Anodenschicht 15.

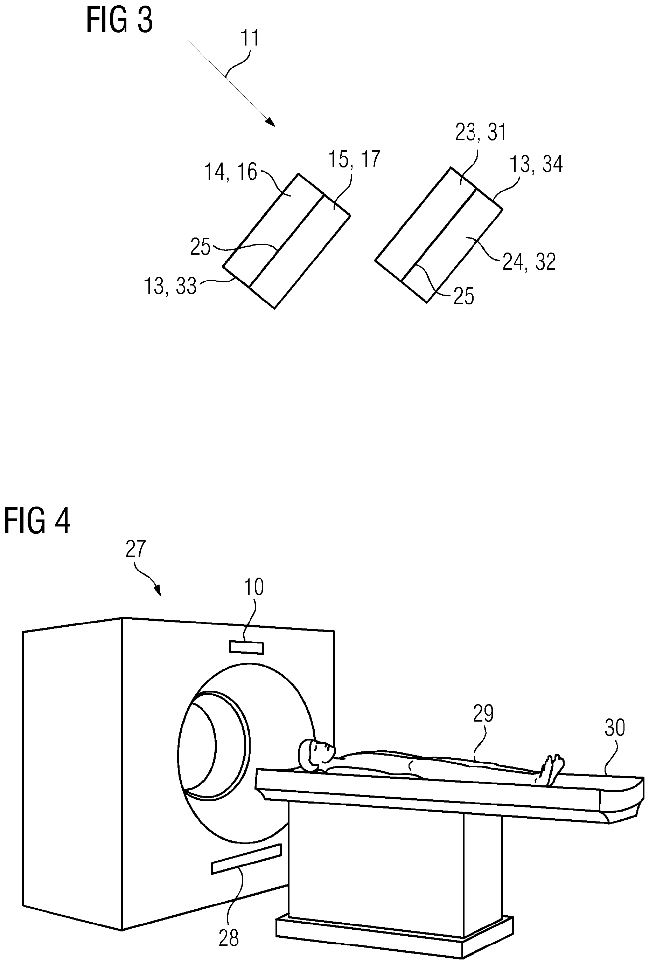

**Fig. 3** zeigt die mehrschichtige Anode 13 in einem dritten Ausführungsbeispiel.

Die mehrschichtige Anode 13 weist eine dem Elektronenstrahl 11 zugewandte dritte Anodenschicht 23 und eine dem Elektronenstrahl 11 abgewandte vierte Anodenschicht 24 auf. Die dritte Anodenschicht 23 weist ein drittes Anodenmaterial 31 zur Generierung der Bremsstrahlung mittels des auftreffenden Elektronenstrahls 11 auf. Die vierte Anodenschicht 24 weist ein viertes Anodenmaterial 32 zur Generierung der weiteren Röntgenstrahlung mittels der Bremsstrahlung auf. Die dritte Anodenschicht 23 und die vierte Anodenschicht 24 grenzen flächig aneinander an. Das zweite Anodenmaterial 17 unterscheidet sich in einer charakteristischen Linie eines Emissionsspektrums von dem vierten Anodenmaterial 32.

Im Vergleich zu dem in Fig. 2 gezeigten Ausführungsbeispiel bilden die erste Anodenschicht 14 und die zweite Anodenschicht 15 eine erste Anodenmikrostruktur 33 und die dritte Anodenschicht 23 und die vierte Anodenschicht 24 eine zweite Anodenmikrostruktur 34. Im Wesentlichen kann sich eine wellenförmige Oberfläche mit periodisch wiederkehrenden Anodenmikrostrukturen mit verschiedenen Emissionsspektren, ergeben, typischerweise unabhängig davon, ob die erste Anodenmikrostruktur 33 und/oder die zweite Anodenmikrostruktur 34 kegelförmig oder rechteckig ausgebildet ist.

In diesem Ausführungsbeispiel stehen die erste Anodenschicht 14, die zweite Anodenschicht 15, die dritte Anodenschicht 23 und die vierte Anodenschicht 24 in einem Winkel von ungefähr 90° zum Elektronenstrahl, wobei der Einfallwinkel des Elektronenstrahls 11 in Hinblick auf die makroskopische Oberfläche kleiner 90° ist. Grundsätzlich ist ein kleinerer oder größerer Winkel denkbar.

Weiterhin ist es denkbar, dass der Elektronenstrahl 11 auf die erste Anodenschicht 14 oder auf die dritte Anodenschicht 23, beispielsweise mittels einer magnetischen Ablenkeinheit der Röntgenröhre 10, ausgerichtet ist. In diesem Fall wird die Bremsstrahlung und die zugehörige weitere Röntgenstrahlung insbesondere mehrheitlich, vorzugsweise zu mehr als 75%, besonders vorteilhafterweise zu mehr als 95%, entweder von der ersten Anodenmikrostruktur 33 oder von der zweiten Anodenmikrostruktur 34 generiert.

**Fig. 4** zeigt eine Röntgeneinrichtung 27 in einem vierten Ausführungsbeispiel. Die Röntgeneinrichtung 27 weist die Röntgenröhre 10 und einen Röntgendetektor 28 auf. Ein Patient 29 ist auf einer Patientenliege 30 gelagert. In diesem Ausführungsbeispiel ist die Röntgeneinrichtung 27 als ein Computertomograph ausgebildet. Alternativ kann die Röntgeneinrichtung 27 ein Teil einer Mammographieeinrichtung oder eines C-Bogen-Angiographie-Systems sein.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgenröhre (10), aufweisend
- einen einen Elektronenstrahl (11) emittierenden Elektronenemitter (12) und
- eine mehrschichtige Anode (13),
- wobei die mehrschichtige Anode (13) eine dem Elektronenstrahl (11) zugewandte erste Anodenschicht (14) und eine dem Elektronenstrahl (11) abgewandte zweite Anodenschicht (15) aufweist,
- wobei die erste Anodenschicht (14) ein erstes Anodenmaterial (16) zur Generierung einer Bremsstrahlung mittels des auftreffenden Elektronenstrahls (11) aufweist,
- wobei die zweite Anodenschicht (15) ein zweites Anodenmaterial (17) zur Generierung einer weiteren Röntgenstrahlung mittels der Bremsstrahlung aufweist,
- wobei das zweite Anodenmaterial (17) ein Konvertermaterial ist,
- wobei die weitere Röntgenstrahlung dadurch monochromatischer ist als die Bremsstrahlung, weil ein Emissionsspektrum der Bremsstrahlung im Vergleich zu einem Emissionsspektrum der weiteren Röntgenstrahlung breitbandiger ist,
- wobei die erste Anodenschicht (14) und die zweite Anodenschicht (15) flächig aneinander angrenzen,
- wobei die erste Anodenschicht (14) gemeinsam mit der zweiten Anodenschicht (15) eine Anodenmikrostruktur (21) bildet,
- wobei die Anodenmikrostruktur (21) entlang einer Oberfläche (22) der mehrschichtigen Anode (13) periodisch wiederkehrend angeordnet ist,
- wobei der Elektronenstrahl (11) auf der Oberfläche (22) in einem Einfallwinkel kleiner 90° auftrifft und
- wobei der Einfallwinkel zwischen 10° und 60° liegt.

2. Röntgenröhre (10) nach Anspruch 1, wobei sich das erste Anodenmaterial (16) in einer charakteristischen Linie eines Emissionsspektrums von dem zweiten Anodenmaterial (17) unterscheidet.

3. Röntgenröhre (10) nach einem der vorhergehenden Ansprüche, wobei das erste Anodenmaterial (16) zumindest ein Material der folgenden Liste aufweist:
- Wolfram,
- Gold.

4. Röntgenröhre (10) nach einem der vorhergehenden Ansprüche, wobei das zweite Anodenmaterial (17) zumindest ein Material der folgenden Liste aufweist:
- Zinn,
- Silber,
- Molybdän,
- Palladium.

5. Röntgenröhre (10) nach einem der vorhergehenden Ansprüche, wobei die mehrschichtige Anode (13) eine dem Elektronenstrahl (11) zugewandte dritte Anodenschicht (23) und eine dem Elektronenstrahl (11) abgewandte vierte Anodenschicht (24) aufweist, wobei die dritte Anodenschicht (23) ein drittes Anodenmaterial (31) zur Generierung der Bremsstrahlung mittels des auftreffenden Elektronenstrahls (11) aufweist, wobei die vierte Anodenschicht (24) ein viertes Anodenmaterial (32) zur Generierung der weiteren Röntgenstrahlung mittels der Bremsstrahlung aufweist, wobei die dritte Anodenschicht (23) und die vierte Anodenschicht (24) flächig aneinander angrenzen und wobei sich das zweite Anodenmaterial (17) in einer charakteristischen Linie eines Emissionsspektrums von dem vierten Anodenmaterial (32) unterscheidet.

6. Röntgenröhre (10) nach Anspruch 5, wobei der Elektronenstrahl (11) auf die erste Anodenschicht (14) oder auf die dritte Anodenschicht (23) ausrichtbar ist.

7. Röntgeneinrichtung (27), aufweisend
- eine Röntgenröhre (10) nach einem der vorhergehenden Ansprüche und
- einen Röntgendetektor (28).

8. Mammographieeinrichtung, aufweisend
- die Röntgeneinrichtung (27) nach Anspruch 7 und
- eine Brustlagerungsvorrichtung.

## Claims

1. X-ray tube (10) having
- an electron emitter (12) emitting an electron beam (11) and
- a multilayer anode (13),
- wherein the multilayer anode (13) has a first anode layer (14) facing the electron beam (11) and a second anode layer (15) facing away from the electron beam (11),
- wherein the first anode layer (14) has a first anode material (16) for generating a braking radiation by means of the incoming electron beam (11)
- wherein the second anode layer (15) has a second anode material (17) for generating a further x-ray radiation by means of the braking radiation,
- wherein the second anode material (17) is converter material,
- wherein as a result the further x-ray radiation is more monochromatic than the braking radiation because an emission spectrum of the braking radiation is wider band compared with an emission spectrum of the further x-ray radiation,
- wherein the first anode layer (14) and the second anode layer (15) adjoin one another in a planar manner,
- wherein the first anode layer (14) together with the second anode layer (15) forms an anode microstructure (21),
- wherein the anode microstructure (21) is arranged in a periodically recurring manner along a surface (22) of the multilayer anode (13),
- wherein the electron beam (11) impinges on the surface (22) at an angle of incidence of less than 90° and
- wherein the angle of incidence can amount in particular to between 10° and 60°.

2. X-ray tube (10) according to claim 1, wherein the first anode material (16) in a characteristic line of an emission spectrum differs from the second anode material (17).

3. X-ray tube (10) according to one of the preceding claims, wherein the first anode material (16) has at least one material from the following list.
- tungsten,
- gold.

4. X-ray tube (10) according to one of the preceding claims, wherein the second anode material (17) has at least one material from the following list.
- tin,
- silver,
- molybdenum,
- palladium.

5. X-ray tube (10) according to one of the preceding claims, wherein the multilayer anode (13) has a third anode layer (23) facing the electron beam (11) and a fourth anode layer (24) facing away from the electron beam (11), wherein the third anode layer (23) has a third anode material (31) for generating the braking radiation by means of the impinging electron beam (11), wherein the fourth anode layer (24) has a fourth anode material (32) for generating the further x-ray radiation by means of the braking radiation, wherein the third anode layer (23) and the fourth anode layer (24) adjoin one another in a planner manner and wherein the second anode material (17) in a characteristic line of an emission spectrum differs from the fourth anode material (32).

6. X-ray tube (10) according to claim 5,
wherein the electron beam (11) is directed at the first anode layer (14) or at the third anode layer (23).

7. X-ray device (27) having
- an x-ray tube (10) according to one of the preceding claims and
- an x-ray detector (28).

8. Mammography device, having
- the x-ray device (27) according to claim 7 and
- a chest support apparatus.

## Revendications

1. Tube (10) à rayons X, comportant :
- un émetteur (12) d'électrons émettant un faisceau (11) d'électrons et
- une anode (13) à plusieurs couches,
- dans lequel l'anode (13) à plusieurs couches a une première couche (14) d'anode tournée vers le faisceau (11) d'électrons et une deuxième couche (15) d'anode non tournée vers le faisceau (11) d'électrons,
- dans lequel la première couche (14) d'anode comporte un premier matériau (16) d'anode pour la création d'un rayonnement de freinage au moyen du faisceau (11) d'électrons incident,
- dans lequel la deuxième couche (15) d'anode comporte un deuxième matériau (17) d'anode pour la création d'un autre rayonnement X au moyen du rayonnement de freinage,
- dans lequel le deuxième matériau (17) d'anode est un matériau de convertisseur,
- dans lequel l'autre rayonnement X est plus monochromatique que le rayonnement de freinage, en ce sens qu'un spectre d'émission du rayonnement de freinage est à bande plus large qu'un spectre d'émission de l'autre rayonnement,
- dans lequel la première couche (14) d'anode et la deuxième couche (15) d'anode sont adjacentes l'une à l'autre en surface,
- dans lequel la première couche (14) d'anode forme conjointement avec la deuxième couche (15) une microstructure (21) d'anode,
- dans lequel la microstructure (21) d'anode est disposée de manière récurrente périodiquement le long d'une surface (22) de l'anode (13) à plusieurs couches,
- dans lequel le faisceau (11) d'électrons arrive sur la surface (22) suivant un angle d'incidence plus petit que 90° et
- dans lequel l'angle d'incidence est compris entre 10° et 60°.

2. Tube (10) à rayons X suivant la revendication 1, dans lequel le premier matériau (16) d'anode se distingue du deuxième matériau (17) d'anode par une raie caractéristique d'un spectre d'émission.

3. Tube (10) à rayons X suivant l'une des revendications précédentes, dans lequel le premier matériau (16) d'anode comporte au moins un matériau de la liste suivante :
- tungstène,
- or.

4. Tube (10) à rayons X suivant l'une des revendications précédentes, dans lequel le deuxième matériau (17) d'anode comporte au moins un matériau de la liste suivante :
- étain,
- argent,
- molybdène,
- palladium.

5. Tube (10) à rayons X suivant l'une des revendications précédentes, dans lequel l'anode (13) à plusieurs couches a une troisième couche (23) d'anode tournée vers le faisceau (11) d'électrons et une quatrième couche (24) d'anode non tournée vers le faisceau (11) d'électrons, dans lequel la troisième couche (23) d'anode comporte un troisième matériau (31) d'anode pour la création du rayonnement de freinage au moyen du faisceau (11) d'électrons incident, dans lequel la quatrième couche (24) d'anode comporte un quatrième matériau (32) d'anode pour la création de l'autre rayonnement X au moyen du rayonnement de freinage, dans lequel la troisième couche (23) d'anode et la quatrième couche (24) d'anode sont voisines l'une de l'autre en surface et dans lequel le deuxième matériau (17) d'anode se distingue du quatrième matériau (32) d'anode par une raie caractéristique d'un spectre d'émission.

6. Tube (10) à rayons X suivant la revendication 5, dans lequel le faisceau (11) d'électrons peut être dirigé sur la première couche (14) d'anode ou sur la troisième couche (23) d'anode.

7. Dispositif (27) à rayons X, comportant
- un tube (10) à rayons X suivant l'une des revendications précédentes, et
- un détecteur (28) de rayons X.

8. Dispositif de mammographie, comportant
- le dispositif (27) à rayons X suivant la revendication 7 et
- un dispositif de positionnement de la poitrine.
